Europäisches Patentamt

European Patent Office

Office européen des brevets

(11) Publication number: **0 217 492**
**A1**

## (12) EUROPEAN PATENT APPLICATION

(21) Application number: **86305253.6**

(22) Date of filing: **08.07.86**

(51) Int. Cl.⁴: **C 07 C 15/44**
**C 07 C 5/333, C 07 C 2/66**

(30) Priority: **26.07.85 US 759191**

(43) Date of publication of application:
**08.04.87 Bulletin 87/15**

(84) Designated Contracting States:
**BE DE FR GB IT NL**

(71) Applicant: **MOBIL OIL CORPORATION**
**150 East 42nd Street**
**New York New York 10017(US)**

(72) Inventor: **Kaeding, Warren William**
**6 Roseberry Court**
**Lawrenceville New Jersey 08648(US)**

(72) Inventor: **Yannich, Philip James, Jr.**
**P.O. Box 53**
**Lake Hill New York 12448(US)**

(72) Inventor: **Klosek, John Michael**
**28 Vernon Street**
**Sewaren New Jersey 07077(US)**

(72) Inventor: **Young, Lewis Brewster**
**26 Pineview Court**
**Skillman New Jersey 08558(US)**

(74) Representative: **Cooper, John Anthony et al,**
**Mobil Court 3 Clements Inn**
**London WC2A 2EB(GB)**

(54) Process for preparing para-divinylbenzene.

(57) A method for preparing para-divinylbenzene involves de-
hydrogenating para-diethylbenzene followed by crystallizing
para-divinylbenzene. The para-diethylbenzene may be prepa-
red by alkylating ethylbenzene with ethylene in the presence
of a para-selective molecular sieve, such as ZSM-5 modified
with an oxide of magnesium and, optionally, an oxide of
phosphorus.

EP 0 217 492 A1

## PROCESS FOR PREPARING
## PARA-DIVINYLBENZENE

This invention relates to a method for preparing para-divinylbenzene by dehydrogenating para-diethylbenzene followed by crystallization of para-divinylbenzene.

Divinylbenzene is a very reactive monomer which may be used as a monomer for preparing polymers or as a crosslinking agent for polymers. Divinylbenzene is presently available as a 40/60 para/meta mixture which is produced by dehydrogenation of the corresponding diethylbenzene mixture. The latter can be produced by alkylation with $AlCl_3$/HCl to give all three diethylbenzene isomers.

Purification of the divinylbenzene mixture by distillation is impractical because of the high temperatures required and its sensitivity to polymerization. Furthermore, these isomers cannot be separated by fractional crystallization when in admixture in such proportions.

In accordance with the invention, there is now provided a method for preparing para-divinylbenzene, the method comprising the steps of: (i) dehydrogenating a feedstock comprising diethylbenzene of which at least 90 percent is para-diethylbenzene to form para-divinylbenzene and para-ethylvinylbenzene; (ii) crystallizing the product of step (i) to form solid crystals of para-divinylbenzene in a mother liquor; and (iii) separating the crystals of para-divinylbenzene from the mother liquor. The diethylbenzene in step (i) comprises at least 90 percent, preferably at least 95 percent, especially at least 98 percent, of the para-isomer.

In accordance with another aspect of the invention, there is provided also a method for preparing para-divinylbenzene, the method comprising the steps of: (iv) alkylating ethylbenzene with ethylene over a para-selective alkylation catalyst comprising a molecular sieve to form a reaction product comprising at least 70 percent by weight of diethylbenzene, at least 90 percent of which is the para-isomer; (v) removing essentially all of the $C_8$ aromatics and hydrocarbons of lesser boiling point from the reaction product of step (iv); and (vi) subjecting the product from step (v) to the method described above.

The para-diethylbenzene-containing feedstock may be supplied from a variety of sources. Preferably, the para-diethylbenzene is prepared by alkylation of ethylbenzene with ethylene over a para-selective molecular sieve catalyst. Such an ethylation reaction is described in U.S. Patent 4,117,024, especially Examples 8 and 9. Preferred ethylation catalysts comprise ZSM-5 modified with an oxide of magnesium and, optionally, an oxide of phosphorus. Such ZSM-5 may be prepared in accordance with the method of U.S. Patent 4,375,458 and may be modified with magnesium oxide in accordance with the procedure described in U.S. Patent 4,447,666.

Since the para-selectivity of ZSM-5 increases with crystal size, very large crystals of ZSM-5 may not need modification at all in order to produce a sufficient proportion of para-diethylbenzene to enable crystallization of para-divinylbenzene after the dehydrogenation step.

When ethylbenzene is alkylated with ethylene, especially in the presence of a catalyst containing ZSM-5, alkylation conditions may include a temperature of 340 to 500°C, a pressure of atmospheric to 200 atmospheres, a weight hourly space velocity of 2 to 2000 and an ethylbenzene/ethylene mole ratio of 1/1 to 20/1.

In addition to ethylation of ethylbenzene over para-selective catalysts, the diethylbenzene for use in the dehydrogenation step of the method of the invention may be derived from a variety of other sources. For example, ethylbenzene may be disproportionated over a para-selective catalyst. U.S. Patent 3,849,508 describes the separation of para-diethylbenzene from a mixture of its isomers by selective adsorption on metal exchanged Y zeolites. High purity para-diethylbenzene has also been prepared by reduction of para-ethylacetophenone - note U.S. Patent 2,390,368.

The dehydrogenation catalyst used in step (i) of the method may be any such catalyst which is capable of achieving the desired

F-3511-L                    - 3 -                    0217492

degree of dehydrogenation without causing an undesired degree of
side reactions, such as isomerization of alkyl groups on the benzene
rings.  Examples of such dehydrogenation catalysts comprise iron
oxide, potassium carbonate, chromium compounds and various promoters,
which do not cause the undesired isomerization of alkyl groups
during the dehydrogenation step.

The following Examples illustrate the invention.

### Example 1

Ethylbenzene is alkylated with ethylene, over a para-
selective zeolite catalyst, to produce high concentrations of the
para isomer in the diethylbenzene product.  A representative example
of results with magnesium modified ZSM-5 zeolite catalyst containing
an alumina binder is shown in Table 1.  The catalyst was regenerated
with hydrogen, overnight, to restore initial activity. Diethylbenzene
is the major product, 75 to 86 percent selectivity.  The para isomer
in the diethylbenzene product ranged from 97.8 percent to 98.8
percent.  Disproportionation of ethylbenzene also gave para-diethylbenzene and benzene.  The benzene by-product can be recycled to make
more ethylbenzene starting material.

Table 1
Alkylation of Ethylbenzene (EB) with Ethylene

Conditions: Temp 425°C; pressure 100 psig; WHSV EB 30.3, $C_2H_4$ 1.7, $H_2$ 0.25; mole ratio $EB/C_2H_4/H_2$ 6.9/1.0/2.9; Mg-ZSM-5 catalyst

| Run | 1 | 3 | 5 | 8 * | 9 | 12 | 16 | 17 | 21 | 23 | 2( |
|---|---|---|---|---|---|---|---|---|---|---|---|
| Time on Stream, hours | 1 | 3 | 20 | 27 | 28 | 32 | 36 | 52 | 60 | 78 | 84 |
| Conversion, EB % | 20.6 | 18.3 | 14.7 | 14.1 | 21.1 | 18.8 | 17.1 | 14.8 | 12.9 | 9.6 | 8. |
| $C_2H_4$ | 71.7 | 73.5 | 68.6 | 55.2 | 76.2 | 70.0 | 68.6 | 62.7 | 54.1 | 38.4 | 35. |
| **Selectivity, Wt %** | | | | | | | | | | | |
| Benzene | 15.0 | 13.7 | 11.6 | 11.5 | 17.1 | 13.5 | 12.5 | 10.6 | 9.5 | 7.2 | 6.9 |
| Toluene | 0.8 | 0.5 | 0.3 | 0.3 | 0.9 | 0.5 | 0.4 | 0.3 | 0.2 | 0.2 | 0 |
| Xylene, ethyltoluene | 3.0 | 2.9 | 2.9 | 3.0 | 3.1 | 2.8 | 2.8 | 2.9 | 3.1 | 3.8 | 4.1 |
| Diethylbenzene (DEB) | 74.2 | 78.7 | 81.9 | 81.9 | 73.7 | 79.0 | 80.2 | 81.9 | 83.8 | 95.4 | 85.5 |
| Other Arom | 2.0 | 1.7 | 1.3 | 1.0 | 2.3 | 1.7 | 1.4 | 1.2 | 1.0 | 0.8 | 0 |
| Gas | 3.5 | 1.3 | 0.7 | 0.8 | 1.3 | 1.2 | 0.9 | 1.5 | 0.6 | 0.6 | 0.5 |
| $C_5-C_9$ | 1.5 | 1.2 | 1.3 | 1.5 | 1.6 | 1.4 | 1.8 | 1.6 | 1.8 | 2.0 | 3.0 |
| TOTAL | 100.0 | 100.0 | 100.0 | 100.0 | 100.0 | 100.0 | 100.0 | 100.0 | 100.0 | 100.0 | 100.0 |
| % Para DEB | 97.8 | 98.1 | 98.5 | 98.5 | 98.0 | 98.4 | 98.5 | 98.5 | 98.6 | 98.8 | 98.8 |

* Catalyst regenerated with hydrogen overnight, 16 hours

F-3511-L

Example 2

The 98+ percent para-diethylbenzene is dehydrogenated to
produce para-ethylvinylbenzene and para-divinylbenzene and hydrogen.
Alkaline dehydrogenation catalysts containing iron oxide, potassium
carbonate, chromium compounds and promoters are used.

Formulations containing iron oxide (45-54 percent),
potassium oxide (43-50 percent), and chromium oxide (2.5-3.5 percent
by weight) are especially effective. Addition of 2 to 5 percent
amounts of other promoters also gives enhanced selectivity. Bismuth
oxide (2-5 percent) increases activity and selectivity. Combinations
of calcium oxide and bismuth oxide or calcium oxide and gallium
oxide give very high selectivities at moderate conversions. In
general catalysts effective for dehydrogenation of p-ethyltoluene to
p-methylstyrene are effective for production of para-divinylbenzene.
Such dehydrogenation catalysts are discussed in U.S. Patents
4,404,123; 4,503,163; and 4,504,594.

A representative dehydrogenation mixture of liquid products
is summarized in Table 2. The alkyl groups are not isomerized
during the dehydrogenation step. The corresponding vinyl products
therefore contain the same isomeric mixtures. By varying the
reaction severity, the ratio of para-ethylvinylbenzene and para-
divinylbenzene can be changed. Higher temperatures (620-630°C) and
contact times plus increased water/hydrocarbon feed ratios favor
para-divinylbenzene. Lower severities (590-620°C) favor para-ethylvinylbenzene.

Small amounts of stabilizers (50-400 ppm), such as
tert-butylcatechol (TBC), are effective for inhibiting polymerization
and decomposition of these reactive monomers during handling and
purification. In addition, insoluble, undesired popcorn polymers
may be prevented by the use of small amounts of $H_2S$ according to
the methods described in U.S. Patent 4,417,084.

## Table 2

### Liquid Dehydrogenation Product

### Temperature 618°C

| Compound | Weight Percent |
|---|---|
| Benzene | 0.2 |
| Toluene | 0.8 |
| Ethylbenzene | 0.6 |
| P-Xylene | 0.2 |
| Ethyltoluene, para | 3.4 |
| meta | 1.3 |
| Diethylbenzene, para | 11.3 |
| , meta | 0.2 |
| Methylstyrene, para | 10.0 |
| , meta | 0.1 |
| Ethylvinylbenzene, para | 22.7 |
| , meta | 0.5 |
| Divinylbenzene, para | 47.7 |
| , meta | 0.7 |
| Other | 0.3 |
| Total | 100.0 |

Additional information on reaction conditions is summarized in Table 3.  In this case, para-divinylbenzene was the major product.

Table 3

Dehydrogenation of P-Diethylbenzene (PDEB) to P-Dinvinylbenzene (PDVB)

Conditions: Temperature 620°C; LSHV 1.1

| Run | 1 | 2 | 3 | 4 | Average Runs 1-4 | 5 | 6 | 7 | 8 | Average Runs 5-8 |
|---|---|---|---|---|---|---|---|---|---|---|
| Time on Stream, Hours | 5 | 7.5 | 24 | 29 | - | 31 | 33 | 50 | 51 | - |
| Conv. PDEB, Weight % | 83 | 86 | 86 | 87 | 85.5 | 86 | 84 | 75 | 75 | 80.0 |
| Steam/PDEB, Weight Ratio | 3.5 | 3.5 | 3.5 | 3.5 | 3.5 | 2 | 2 | 2 | 2 | 2 |
| **Products, Weight %** | | | | | | | | | | |
| $H_2$, $C_2$-$C_7$ | 13.0 | 14.7 | 14.6 | 15.1 | 14.4 | 17.1 | 13.9 | 9.1 | 9.0 | 12.3 |
| PDEB | 16.7 | 13.9 | 14.3 | 13.4 | 14.6 | 13.8 | 16.0 | 24.8 | 24.8 | 19.8 |
| PEVB | 26.8 | 25.6 | 25.9 | 25.7 | 26.0 | 28.8 | 30.4 | 30.7 | 31.0 | 30.2 |
| PDVB | 43.4 | 45.6 | 45.1 | 45.6 | 44.9 | 40.2 | 39.4 | 35.2 | 35.0 | 37.5 |
| $C_{11}$+ | 0.1 | 0.2 | 0.1 | 0.2 | 0.1 | 0.1 | 0.3 | 0.2 | 0.2 | 0.2 |
| Total | 100.0 | 100.0 | 100.0 | 100.0 | 100.0 | 100.0 | 100.0 | 100.0 | 100.0 | 100.0 |

F-3511-I

0217492

Separation, purification and recycle are important aspects of a useful process. In this instance, the ability to start with 98+ percent para-diethylbenzene provides some unique opportunities. Virtually all of the meta isomers are absent. After the dehydrogenation step, the desired para-divinylbenzene is present in relatively high concentration and crystallizes as pure white needles, mp 31-32°C. The substantial presence of meta isomers prevents this. This very active monomer, para-divinylbenzene, is separated and purified by reducing the temperature.

This is in sharp contrast with the present commercial process for producing divinylbenzene. This product contains approximately a 2/1 meta/para mixture. Heating is used to remove as much of the lower boiling products as possible. It is, however, impractical to provide a product with greater than 80 percent divinylbenzene purity. These monomers are simply too reactive to survive extended heating.

The present commercial product is, therefore, composed of a meta/para mixture determined by the initial composition of the diethylbenzene. It contains some unreacted diethylbenzene, ethylvinylbenzene and divinylbenzene.

Crystallization of the para-divinylbenzene may be carried out in the presence or absence of added liquids. For example, judicious use of solvents may also be used to induce and promote crystallization. Pentane or petroleum ethers are effective and may be easily separated by distillation from the mother liquor.

If desired, the mother liquor remaining after removal of pure para-divinylbenzene may be distilled under vacuum to remove the para-ethylstyrene (PES) as a product. Alternatively, it may be recycled with the para-diethylbenzene starting material and converted to more para-divinylbenzene.

CLAIMS:

1. A method for preparing para-divinylbenzene, comprising the steps of:

    (i) dehydrogenating a feedstock comprising diethylbenzene of which at least 90 percent is para-diethylbenzene to form para-divinylbenzene and para-ethylvinylbenzene;

    (ii) crystallizing the product of step (i) to form solid crystals of para-divinylbenzene in a mother liquor; and

    (iii) separating the crystals of para-divinylbenzene from the mother liquor.

2. A method according to Claim 1, wherein at least 95 percent of the diethylbenzene is the para-isomer.

3. A method according to Claim 1 or Claim 2, wherein at least 98 percent of the diethylbenzene is the para-isomer.

4. A method according to any one of Claims 1 to 3, wherein the dehyrogenation step (i) is carried out at 620-630°C.

5. A method according to any one of Claims 1 to 4, wherein the dehydrogenation catalyst of step (i) contains 45-54 weight percent iron oxide, 43-50 weight percent potassium oxide and 2.5-3.5 weight percent chromium oxide.

6. A method according to any one of Claims 1 to 5, wherein at least 75 percent of the para-diethylbenzene is converted in dehydrogenation step (i).

7. A method for preparing para-divinylbenzene, comprising the steps of:

(iv) alkylating ethylbenzene with ethylene over a para-selective alkylation catalyst comprising a molecular sieve to form a reaction product comprising at least 70 percent by weight of diethylbenzene, at least 90 percent of which is the para-isomer;

(v) removing essentially all of the $C_8$ aromatics and hydrocarbons of lesser boiling point from the reaction product of step (iv); and

(vi) subjecting the product from step (v) to the method of any one of Claims 1 to 6.

8. A method according to Claim 7, wherein the molecular sieve is zeolite ZSM-5.

9. A method according to Claim 8, wherein the ZSM-5 is modified with magnesium oxide.

10. A method according to Claim 9, wherein the ZSM-5 is modified also with phosphorus oxide.

9217H/0615H

**European Patent Office**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | US-A-3 715 408  (BROWN et al.)<br>*  Column  2,  paragraphs  3-5;<br>claims 1,5,6 *<br>--- | 1,7 | C 07 C   15/44<br>C 07 C    5/333<br>C 07 C    2/66 |
| D,X | US-A-4 117 024  (KAEDING)<br>* Column 1, lines 38-40; claims *<br>--- | 7-10 | |
| D,X | US-A-4 504 594  (CHU)<br>* Claims *<br>--- | 5 | |
| A | US-A-3 360 579  (G.J. HILLS et al.)<br>* Column 1 *<br>----- | | |

**TECHNICAL FIELDS SEARCHED (Int Cl 4)**

C 07 C   15/00
C 07 C    7/00
C 07 C    5/00
C 07 C    2/00

The present search report has been drawn up for all claims

| Place of search | Date of completion of the search | Examiner |
|---|---|---|
| THE HAGUE | 31-10-1986 | VAN GEYT J.J.A. |